# EUROPEAN PATENT APPLICATION

(11) **EP 4 825 985 A1**
(43) Date of publication of application: **21.10.2026**
(21) Application number: 25171337.6
(22) Date of filing: 17.04.2025
(51) Int. Cl.: C12Q 1/6895, C12Q 1/6844, C12Q 1/70

(54) **NOVEL LOOP MEDIATED ISOTHERMAL AMPLIFICATION (LAMP) PRIMERS FOR DETECTION OF IMPORTANT SUGAR BEET PATHOGENS**

(71) Applicant: KWS SAAT SE & Co. KGaA, 37574 Einbeck (DE)
(72) Inventor: Purahong, Witoon, 37574 Einbeck (DE); Krüger, Kerstin, 37574 Einbeck (DE)
(74) Representative: Eisenführ Speiser

(57) **Abstract**

The present invention pertains to the field of molecular diagnostics, specifically focusing on the application of nucleic acid-based detection methods for plant pathogens. It relates the use of loop-mediated isothermal amplification (LAMP) primer sets to identify specific pathogens affecting Beta spp., particularly sugar beet (Beta vulgaris), including yellowing viruses and fungal pathogens. The invention finds applications in agricultural biotechnology, plant disease management, and in-field diagnostic testing, offering advancements in the rapid and accurate detection of plant pathogens for improved crop protection and management strategies.

## Description

### Technical Field

The present invention relates to the field of molecular diagnostics, specifically focusing on the application of nucleic acid-based detection methods for plant pathogens. It relates the use of loop-mediated isothermal amplification (LAMP) primer sets to identify specific pathogens affecting Beta spp., particularly sugar beet (*Beta vulgaris*), including yellowing viruses and fungal pathogens. The invention finds applications in agricultural biotechnology, plant disease management, and in-field diagnostic testing, offering advancements in the rapid and accurate detection of plant pathogens for improved crop protection and management strategies.

### Background

Cercospora leaf spot (CLS), caused by the fungal pathogen *Cercospora beticola,* is a significant disease affecting sugar beet crops worldwide. The disease leads to substantial yield losses due to reduced photosynthetic capacity and increased management costs. Traditional management approaches for CLS rely heavily on fungicide applications, but overuse has led to increased resistance in many *Cercospora beticola* populations. As a result, there is a pressing need for early and accurate detection methods to manage and mitigate the spread of this pathogen effectively.

In addition to *Cercospora beticola,* sugar beet crops are also threatened by yellowing viruses, including beet chlorosis virus (BChV), Beet mild yellowing virus (BMYV), and Beet yellows virus (BYV). Current detection methods for these viruses typically require samples to be sent to laboratories for analysis, which is both time-consuming and costly, limiting the potential for in-field diagnostics and immediate action.

Loop-Mediated Isothermal Amplification (LAMP) is a nucleic acid amplification technique that enables rapid and specific DNA replication at a constant temperature, typically between 60 and 65°C. Unlike conventional PCR, which requires thermal cycling, LAMP uses a strand-displacing DNA polymerase and a specially designed set of primers to facilitate continuous amplification without high-temperature denaturation.

The reaction is initiated by a set of four to six primers that recognize six to eight distinct regions of the target DNA sequence. The key primers include forward and backward outer primers (F3 and B3), which initiate DNA synthesis, and forward and backward inner primers (FIP and BIP), which play a central role in amplifying the DNA. Additional loop primers (LoopF and LoopB) can further accelerate the reaction by creating additional priming sites. The enzyme used in LAMP is a DNA polymerase with a strong strand displacement activity, allowing DNA synthesis to proceed efficiently without requiring repeated heating and cooling cycles.

The amplification process begins when the FIP primer binds to the target DNA and initiates synthesis. The outer F3 primer then binds to a nearby sequence and displaces the newly synthesized strand, forming a self-priming looped structure. A similar process occurs on the opposite strand with BIP and B3 primers, leading to the formation of a dumbbell-shaped DNA molecule. This structure enables continuous amplification through a self-sustaining loop mechanism, resulting in the rapid production of large amounts of DNA. The unique design of the reaction allows exponential DNA amplification, forming highly branched DNA products with multiple loops.

LAMP provides an efficient and highly sensitive method for DNA detection, capable of amplifying even a few copies of target DNA, usually in less than 90 minutes. The reaction produces large quantities of DNA, which can be detected through various means, including visual turbidity caused by magnesium pyrophosphate precipitation, colorimetric dyes such as SYBR Green or hydroxy naphthol blue, and fluorescence-based detection methods. Unlike conventional PCR, which requires gel electrophoresis, LAMP products can often be analyzed by simple visual inspection, making it particularly useful for point-of-care diagnostics and field applications.

However, while LAMP is a powerful tool for rapid DNA amplification, it does have several potential disadvantages that can limit its application in certain scenarios. These drawbacks primarily stem from its complexity, specificity requirements, and detection limitations.

One of the main challenges with LAMP is its primer design complexity. Unlike conventional PCR, which typically uses just two primers, LAMP requires a carefully designed set of four to six primers that recognize multiple regions of the target sequence. This intricate primer system makes assay development more challenging and requires extensive optimization. Designing effective primers can be particularly difficult for organisms with high genetic variability, as mutations in the target regions may disrupt primer binding and lead to inconsistent results.

A study by Shrestha et al. (2020) (Shresta et al., Rapid detection of Cercospora beticola in sugar beet and mutations associated with fungicide resistance using LAMP or probe-based qPCR, Plant Disease (2020), 104, 1654-1661), relates to LAMP assays for detecting *Cercospora beticola.* However, these existing LAMP primers are limited as they only consist of four primers, necessitating longer incubation times of up to 90 minutes for effective detection. This delay can hinder timely decision-making in field conditions where rapid detection is critical.

The invention solves these problems by providing a novel primer set for loop-mediated isothermal amplification (LAMP) assays, enabling the rapid, sensitive, and specific detection of both yellowing viruses and *Cercospora beticola* directly in the field. By incorporating six primers, including novel LoopF and LoopB primers, the invention reduces detection time significantly compared to existing methods, facilitating early intervention and improved disease management strategies.

### Summary of the invention

The above mentioned problem is solved in a first aspect by providing a primer set for detecting the presence or absence of a *Beta spp.* specific, preferably a *Beta vulgaris* specific, pathogen, suitable for use in a loop-mediated isothermal amplification (LAMP) assay, preferably for the simultaneously detecting at least two *Beta spp.* specific, preferably a *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola,* wherein each of said at least one primer set comprises a forward primer, a reverse primer, a forward inner primer, a reverse inner primer, a loop forward primer and a loop reverse primer, wherein the primer sets are selected from: (i) a primer set for detecting BChV, comprising one primer each from the six groups selected from (i1) SEQ ID NO: 1 and 2, (i2) SEQ ID NO: 3 and 4, (i3) SEQ ID NO: 5 and 6, (i4) SEQ ID NO: 7 and 8, (i5) SEQ ID NO: 9 and 11, (i6) SEQ ID NO: 12 and 13, and/or (ii) a primer set for detecting BMYV, comprising one primer each from the six groups selected from (ii1) SEQ ID NO: 14, (ii2) SEQ ID NO: 15, (ii3) SEQ ID NO: 16 and 17, (ii4) SEQ ID NO: 18 and 19, (ii5) SEQ ID NO: 20, (ii6) SEQ ID NO: 21, and/or (iii) a primer set for detecting BYV, comprising one primer each from the six groups selected from (iii1) SEQ ID NO: 22 and 23, (iii2) SEQ ID NO: 24 and 25, (iii3) SEQ ID NO: 26 and 27, (iii4) SEQ ID NO: 28 and 29, (iii5) SEQ ID NO: 30 and 31, (iii6) SEQ ID NO: 32 and 33, and/or (iv) a primer set for detecting *Cercospora beticola,* comprising one primer each from the six groups selected from (iv1) SEQ ID NO: 34 and 35, (iv2) SEQ ID NO: 36 and 37, (iv3) SEQ ID NO: 38 and 39, (iv4) SEQ ID NO: 40 and 41, (iv5) SEQ ID NO: 42 and 43, and/or (iv6) SEQ ID NO: 44 and 45.

One embodiment relates to the primer set of the first aspect comprising at least two, three, or all four primer sets of (i), (ii), (iii), or (iv) in combination with each other.

One embodiment relates to the primer set according to the first aspect or embodiment thereof, wherein the primer set for detecting BChV comprises the set of sequences as defined by SEQ ID NO: 2, 4, 6, 8, 11 and 13, and/or the primer set for detecting BMYV comprises the set of sequences as defined by SEQ ID NO: 14, 15, 17, 18, 20 and 21, and/or the primer set for detecting BYV comprises the set of sequences as defined by SEQ ID NO: 22, 24, 26, 28, 30 and 32, and/or wherein primer set for detecting *Cercospora beticola* comprises the set of sequences as defined by SEQ ID NO: 35, 37, 39, 41, 43 and 45.

One embodiment relates to the primer set according to the first aspect or any embodiment thereof, wherein at least one primer, preferably at least one forward inner or at least one reverse primer, comprises at least one label, preferably a 5'-label, preferably wherein the label is selected from the group of biotin, FITC and FAM.

A second aspect relates to a detection kit for detecting the presence or absence of at least one *Beta spp.* specific, preferably a *Beta vulgaris* specific, pathogen, wherein the kit comprises at least one primer set according to the first aspect or any embodiment thereof, and optionally at least one suitable buffer and/or at least one suitable solution, preferably a lysis buffer.

One embodiment relates to the detection kit according to the second embodiment, wherein each primer or primer set is contained in one container or compartment in a lyophilized form, or wherein each primer or primer set is composed of a solution or mixed solution.

One embodiment relates to the detection kit according to the second embodiment, wherein the detection kit is configured for *in situ* use in a field, wherein the kit comprises (i) at least one primer set according to any one of claims 1 to 4; (ii) at least one test cartridge, optionally including at least one sample vial comprising: a reaction chamber or cartridge configured to receive a sample; (iii) at least one enzyme, optionally immobilized or provided in or with at least one buffer, for performing a LAMP assay on a sample; (iv) at least one portable heating system, preferably heat block, wherein the at least one portable heating system achieves a temperature of up to 55°C, preferably up to 60°C, more preferably up to 65°C, most preferably at least 65°C; (v) optionally: at least one high performance power bank; (vi) optionally: at least one suitable chemical or mechanical reagent; and (vii) optionally: at least one micropipette.

A third aspect relates to a method of detecting the presence or absence of a *Beta spp.* specific, preferably a *Beta vulgaris* specific, pathogen, suitable for use in a loop-mediated isothermal amplification (LAMP) assay, preferably for the simultaneously detecting at least two *Beta spp.* specific, preferably a *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola,* the method comprising: (i) obtaining and thus providing a sample potentially comprising genetic information of at least one *Beta spp.* specific, preferably a *Beta vulgaris* specific, pathogen; (ii) optionally: mechanically pre-treating the sample; (iii) conducting a LAMP assay with said sample using at least one primer set as defined in the first aspect of any embodiment thereof, or using a detection kit as defined in the second aspect or any embodiment thereof; (iv) obtaining and optionally evaluating at least one test result from the LAMP assay.

One embodiment relates to the method according to the third aspect, further comprising the steps of; (v) identifying at least one *Beta spp.* plant, preferably a *Beta vulgaris* plant tested positive for at least one *Beta spp.* specific pathogen; and (vi) optionally: identifying and optionally applying a pathogen specific treatment.

One embodiment relates to the method according to the third aspect or the embodiment thereof, wherein the method is at least partially, preferably completely, conducted *in situ* or in a non-laboratory environment.

One embodiment relates to the method according to the third aspect or any embodiment thereof, wherein the method is a multiplexing isothermal detection method for detecting at least two, preferably at least three, more preferably for or more than four *Beta spp.* specific, preferably *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola.*

One embodiment relates to the method according to the third aspect or any embodiment thereof, wherein step (iii) comprises a step of lysis.

One embodiment relates to the method according to the third aspect or any embodiment thereof, wherein the at least one test result is available in less than one hour, preferably in less than 45 min, more preferably in less than 30 min and most preferably in less than 15 min.

A fourth aspect relates to a use of at least on primer set according to the first aspect or any embodiment thereof and/or a use of a detection kit according to the second aspect or any embodiment thereof for detecting the presence or absence of a *Beta spp.* specific, preferably a *Beta vulgaris* specific, pathogen, suitable for use in a loop-mediated isothermal amplification (LAMP) assay, preferably for the simultaneously detecting at least two *Beta spp.* specific, preferably a *Beta vulgaris* specific, pathogens.

One embodiment relates to the use of the fourth aspect, wherein the use is an *in situ* use conducted in a non-laboratory environment.

### Brief Description of Drawings

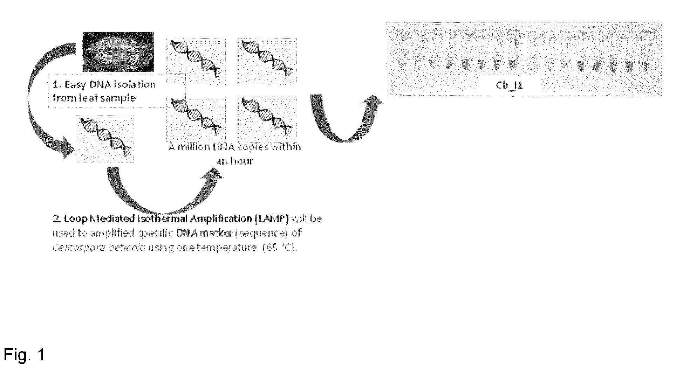

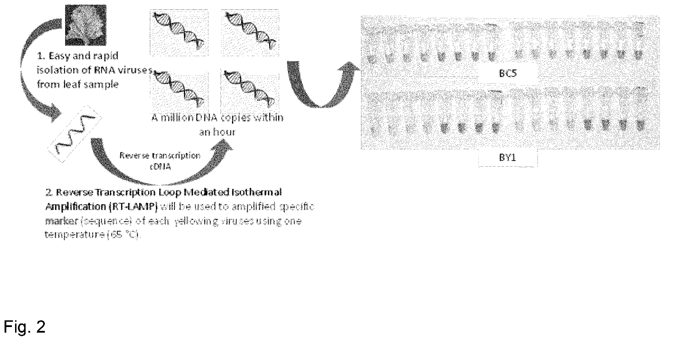

Figure (Fig. 1): Detection limit results from loop mediated isothermal amplification (LAMP) primer sets.
   Incubation times for BY1 (BYV) and Cb_I1 = 30 min and BChV = 45 min. Concentrations of *C. beticola =* 1 to 10⁻⁶ ng/µl pure DNA of *C. beticola* strains cb150 and cb266. Concentrations of yellowing viruses = 1 to 10⁻⁶ ng/µl plant-virus mixed RNA of BChV (strains BC17 and BC18) and BYV (BY 76 and BY77). The last tube is negative control (PCR water). Plant extract negative controls as well as the primer set BM3new (BMYV) were tested separately.
Figure (Fig. 2): Detection limit results from reverse transcribed loop mediated isothermal amplification (RT-LAMP) primer sets.
   Incubation times for BY1 (BYV) and Cb_I1 = 30 min and BChV = 45 min. Concentrations of *C. beticola =* 1 to 10⁻⁶ ng/µl pure DNA of *C. beticola* strains cb150 and cb266. Concentrations of yellowing viruses = 1 to 10⁻⁶ ng/µl plant-virus mixed RNA of BChV (strains BC17 and BC18) and BYV (BY 76 and BY77). The last tube is negative control (PCR water). Plant extract negative controls as well as the primer set BM3new (BMYV) were tested separately.
Figure 3: Successful validation of LAMP tests based on LyoPrime WarmStart fluorescent LAMP/RT-LAMP Mix (with UDG).
   Yellowing viruses detection using LAMP based on LyoPrime WarmStart fluorescent LAMP/RT-LAMP Mix (with UDG) and Milenia HybriDetect (C = Control, BC = BChV, BM = BMYV and BY = BYV).
Figure 4: LAMP tests based on LyoPrime WarmStart fluorescent LAMP/RT-LAMP Mix (with UDG) for detection of *Cercospora.*
   The (green) triangle indicates the positive test result for a probe with early stage Cercospora infection, demonstrating high test sensitivity.
Figure 5: Representative LAMP assay tool kit and experimental workflow.

### Brief Description of Sequences

| **Seq ID NO** | **Primer name** | **Seq ID NO** | **Primer name** |
|---|---|---|---|
| 1 | F3_BC1 x1 | 24 | B3_BY1 z2 |
| 2 | F3_BC5 x1 | 25 | B3_BY3 z2 |
| 3 | B3_BC1 x2 | 26 | FIP_BY1 z3 |
| 4 | B3_BC5 x2 | 27 | FIP_BY3 z3 |
| 5 | FIP_BC4 x3 | 28 | BIP_BY1 z4 |
| 6 | FIP_BC5 x3 | 29 | BIP_BY3 z4 |
| 7 | BIP_BC1 x4 | 30 | LoopF_BY1 z5 |
| 8 | BIP_BC5 x4 | 31 | LoopF_BY3 z5 |
| 9 | LoopF_BC1 x5 | 32 | LoopB_BY1 z6 |
| 10 | LoopF_BC1_2 | 33 | LoopB_BY3 z6 |
| 11 | LoopF_BC5 | 34 | F3_CB_C2 w1 |
| 12 | LoopB_BC1 x6 | 35 | F3_CB_I1 w1 |
| 13 | LoopB_BC5 x6 | 36 | B3_CB_C2 w2 |
| 14 | F3_BM1 y1 | 37 | B3_CB_I1 w2 |
| 15 | B3_BM1 | 38 | FIP_CB_C2 w3 |
| 16 | FIP_BM1 y3 | 39 | FIP_CB_I1 w3 |
| 17 | FIP_BM3 y3 | 40 | BIP_CB_C2 w4 |
| 18 | BIP_BM1 y4 | 41 | BIP_CB_I1 w4 |
| 19 | BIP_BM2 y4 | 42 | LoopF_CB_C2 w5 |
| 20 | LoopF_BM1 y5 | 43 | LoopF_CB_I1 w5 |
| 21 | LoopB_BM1 y6 | 44 | LoopB_CB_C2 w6 |
| 22 | F3_BY1 z1 | 45 | LoopB_CB_I1 w6 |
| 23 | F3_BY3 z1 | | |

### Detailed Description

Sugar beet production is increasingly threatened by viral and fungal pathogens, including yellowing viruses (*Beet Mild Yellowing Virus* (BMYV), *Beet Yellow Virus* (BYV), and *Beet Chlorosis Virus* (BChV)) and the fungal pathogen *Cercospora beticola.* These pathogens cause significant yield losses and reduce crop quality, necessitating timely and accurate detection methods for effective disease management. However, existing diagnostic techniques often require complex laboratory procedures, expensive equipment, or extended processing times, limiting their practical use in field conditions where rapid decision-making is crucial.

The present invention addresses this urgent need by providing novel Loop-Mediated Isothermal Amplification (LAMP) primers specifically designed for the rapid and efficient detection of these sugar beet pathogens. These primers enable the development of highly sensitive and specific LAMP assays, which can be performed in the field without the need for sophisticated laboratory infrastructure. By facilitating real-time pathogen detection, the invention significantly improves plant pathogen diagnostics, integrated plant protection strategies, disease monitoring, seed quality assessment, in-field agronomic consultancy, and plant breeding programs. The availability of such robust and field-deployable diagnostic tools empowers growers and agricultural professionals to implement timely disease management strategies, ultimately enhancing crop protection and productivity.

According to a first aspect, the problem is solved by providing a primer set for detecting the presence or absence of a *Beta spp.* specific, preferably a *Beta vulgaris* specific, pathogen, suitable for use in a loop-mediated isothermal amplification (LAMP) assay, preferably for the simultaneously detecting at least two *Beta spp.* specific, preferably a *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola,* wherein each of said at least one primer set comprises a forward primer, a reverse primer, a forward inner primer, a reverse inner primer, a loop forward primer and a loop reverse primer, wherein the primer sets are selected from: (i) a primer set for detecting BChV, comprising one primer each from the six groups selected from (i1) SEQ ID NO: 1 and 2, (i2) SEQ ID NO: 3 and 4, (i3) SEQ ID NO: 5 and 6, (i4) SEQ ID NO: 7 and 8, (i5) SEQ ID NO: 9 and 11, (i6) SEQ ID NO: 12 and 13, and/or (ii) a primer set for detecting BMYV, comprising one primer each from the six groups selected from (ii1) SEQ ID NO: 14, (ii2) SEQ ID NO: 15, (ii3) SEQ ID NO: 16 and 17, (ii4) SEQ ID NO: 18 and 19, (ii5) SEQ ID NO: 20, (ii6) SEQ ID NO: 21, and/or (iii) a primer set for detecting BYV, comprising one primer each from the six groups selected from (iii1) SEQ ID NO: 22 and 23, (iii2) SEQ ID NO: 24 and 25, (iii3) SEQ ID NO: 26 and 27, (iii4) SEQ ID NO: 28 and 29, (iii5) SEQ ID NO: 30 and 31, (iii6) SEQ ID NO: 32 and 33, and/or (iv) a primer set for detecting *Cercospora beticola,* comprising one primer each from the six groups selected from (iv1) SEQ ID NO: 34 and 35, (iv2) SEQ ID NO: 36 and 37, (iv3) SEQ ID NO: 38 and 39, (iv4) SEQ ID NO: 40 and 41, (iv5) SEQ ID NO: 42 and 43, and/or (iv6) SEQ ID NO: 44 and 45.

Advantageously, the primer set is designed to detect at least one pathogen from a group consisting of beet chlorosis virus (BChV), Beet mild yellowing virus (BMYV), Beet yellows virus (BYV), and the fungal pathogen *Cercospora beticola.* Each primer set comprises six primers: a forward primer, a reverse primer, a forward inner primer, a reverse inner primer, a loop forward primer, and a loop reverse primer. The inclusion of LoopF and LoopB primers enhances the assay's sensitivity and reduces the incubation time required for detection.

The primer sets are selected from specific sequences associated with each pathogen, ensuring high specificity and sensitivity. For example, the primer set for detecting BChV is defined by specific SEQ ID numbers for each of the six primers.

In certain embodiments, the LAMP assay may be performed as a Reverse Transcription Loop-Mediated Isothermal Amplification (RT-LAMP) assay, enabling the detection of RNA viruses or other RNA-based targets. In this configuration, the RT-LAMP assay incorporates a reverse transcription step within the same reaction tube, allowing for the simultaneous conversion of RNA into complementary DNA (cDNA) and its subsequent amplification under isothermal conditions. This is achieved using reverse transcriptase, which operates alongside strand-displacing DNA polymerase, eliminating the need for separate reverse transcription and amplification steps.

One embodiment, there is provided a primer set comprising at least two, three, or all four primer sets of (i), (ii), (iii), or (iv) in combination with each other.

In another embodiment, there is provided a primer set, wherein the primer set for detecting BChV comprises the set of sequences as defined by SEQ ID NO: 2, 4, 6, 8, 11 and 13, and/or the primer set for detecting BMYV comprises the set of sequences as defined by SEQ ID NO: 14, 15, 17, 18, 20 and 21, and/or the primer set for detecting BYV comprises the set of sequences as defined by SEQ ID NO: 22, 24, 26, 28, 30 and 32, and/or wherein primer set for detecting *Cercospora beticola* comprises the set of sequences as defined by SEQ ID NO: 35, 37, 39, 41, 43 and 45.

In yet another embodiment, there is provided a primer set, wherein at least one primer, preferably at least one forward inner or at least one reverse primer, comprises at least one label, preferably a 5'-label, preferably wherein the label is selected from the group of biotin, FITC and FAM.

In some embodiments, dual-labeled primers may be used to create FRET (Förster Resonance Energy Transfer)-based probes, allowing highly specific signal generation and reducing false-positive results in fluorescence-based detection systems.

In some embodiments, the labeled primer set may be specifically optimized for lateral flow assays (LFAs), where biotin- or FITC-labeled amplicons can be detected using test strips, eliminating the need for specialized detection equipment in field applications.

In some embodiments, multiplex detection strategies may utilize differentially labeled primers, each targeting distinct pathogens, allowing for the simultaneous detection of multiple sugar beet pathogens in a single reaction through differential fluorescence readout or colorimetric detection.

In some embodiments, the primer set may be adapted for automated detection systems, where labeled amplicons are detected using microfluidic devices or portable fluorescence readers, enhancing scalability and high-throughput diagnostic applications.

In some embodiments, the labeled primers may be compatible with isothermal amplification techniques beyond LAMP, including recombinase polymerase amplification (RPA) and nucleic acid sequence-based amplification (NASBA), broadening their applicability for nucleic acid-based pathogen detection.

In some embodiments, the labeled primers may be designed for hybridization-based detection, where labeled probes bind to complementary amplified sequences, enabling downstream microarray-based pathogen identification or integration into next-generation sequencing workflows for high-resolution disease diagnostics.

In some embodiments, the primer set may include labels that provide colorimetric, chemiluminescent, or electrochemical signal output, making them adaptable to biosensor-based detection platforms.

A second aspect, there is provided a detection kit for detecting the presence or absence of at least one *Beta spp.* specific, preferably a *Beta vulgaris* specific, pathogen, wherein the kit comprises at least one primer set according to the present invention, and optionally at least one suitable buffer and/or at least one suitable solution, preferably a lysis buffer.

In some embodiments, the detection kit is designed for efficient in-field use, offering a comprehensive solution for the rapid detection of sugar beet pathogens. The kit may include at least one LAMP primer set along with various essential and optional components required for sample preparation, nucleic acid amplification, and result interpretation.

In some embodiments, the detection kit comprises primer sets specifically designed for the detection of sugar beet pathogens, including but not limited to yellowing viruses (*BMYV, BYV, BChV*) and *Cercospora beticola.* These primers may be formulated to provide high sensitivity and specificity in LAMP-based assays, ensuring accurate pathogen identification.

In some embodiments, the detection kit includes lysis buffers optimized for different pathogen types. For RNA viruses, the lysis buffer may comprise guanidine hydrochloride, Triton X-100, and a Tris-based solution to facilitate effective viral RNA extraction, but not limited thereof. For fungal DNA, the lysis buffer may comprise NaCl, polyvinylpyrrolidone (PVP), Tween, and a Tris-based solution, ensuring efficient fungal DNA extraction, but not limited thereof.

In some embodiments, the primers within the detection kit may be provided in a lyophilized form to enhance stability and shelf life, making them suitable for extended storage and field deployment. Alternatively, the primers may be supplied in a solution or mixed solution form for immediate use, facilitating convenient field applications.

In some embodiments, the detection kit may further include a micropipette for precise sample handling and reagent transfer, improving accuracy and reproducibility in field diagnostic testing.

In some embodiments, the detection kit is configured to accommodate mechanical pretreatment of samples. Sample processing may be performed using a plant grinding machine for efficient tissue disruption. Alternatively, plant material may be placed into a pre-filled lysis buffer bag, where it can be macerated using a pen cap or the end of a pen body, providing a simple, equipment-free method for sample preparation in field conditions.

In some embodiments, the detection kit may be applicable to both direct and indirect sample analysis. Direct samples may include sugar beet leaves, roots, and seeds, which are commonly tested for pathogen presence. Indirect samples, such as soil, insect vectors (e.g., aphids), weeds, sugar beet flowers, and other plant species, may also be analyzed to assess potential reservoirs and transmission sources of pathogens. This expanded sampling capability may be particularly advantageous for epidemiological studies and monitoring pathogen spread.

In some embodiments, the detection kit may be designed for field-ready use, eliminating the requirement for laboratory infrastructure. This feature may enhance accessibility for agronomists, plant pathologists, and farmers, enabling real-time pathogen detection and monitoring.

In some embodiments, the detection kit may offer a rapid and cost-effective testing solution, allowing for timely decision-making in disease control strategies and reducing crop losses. The streamlined workflow of the kit may minimize reliance on complex laboratory procedures and specialized personnel, making it suitable for broad agricultural applications.

In some embodiments, the detection kit may be utilized beyond routine plant pathogen diagnostics and integrated into surveillance programs. The ability to analyze indirect samples may facilitate vector monitoring and the identification of alternative pathogen sources, contributing to improved disease management strategies.

In one embodiment, there is thus provided a detection kit, wherein each primer or primer set is contained in one container or compartment in a lyophilized form, or wherein each primer or primer set is composed of a solution or mixed solution.

In another embodiment, there is provided a detection kit according to the present invention, wherein the detection kit is configured for *in situ* use in a field, wherein the kit comprises (i) at least one primer set according to present invention; (ii) at least one test cartridge, optionally including at least one sample vial comprising: a reaction chamber or cartridge configured to receive a sample; (iii) at least one enzyme, optionally immobilized or provided in or with at least one buffer, for performing a LAMP assay on a sample; (iv) at least one portable heating system, preferably heat block, wherein the at least one portable heating system achieves a temperature of at least 50°C, preferably at least 60°C, more preferably at least 65°C; (v) optionally: at least one high performance power bank; (vi) optionally: at least one suitable chemical or mechanical reagent; and (vii) optionally: at least one micropipette.

In some embodiments, the detection kit may include a portable heating system (heat block) capable of reaching at least 50°C, preferably at least 60°C, more preferably at least 65°C to support optimal reaction conditions for the LAMP assay. The heating system may be powered by a high-performance power bank, allowing operation in remote locations without the need for an external power source.

In some embodiments, the heating system is configured to provide uniform heat distribution across all reaction tubes to minimize variations in amplification efficiency. It may feature rapid heating and temperature stabilization capabilities, reducing the time required to reach the desired reaction temperature and ensuring consistent assay performance.

In some embodiments, the heating system is designed to operate in field conditions, making it compact, lightweight, and portable. It is preferably powered by a high-performance rechargeable power bank, allowing operation in remote locations without access to conventional power sources. The power system may support extended run times, enabling multiple assays to be conducted in a single field session.

In some embodiments, the heating system may have a modular or multi-well format, accommodating multiple samples simultaneously to increase throughput. The device may include an integrated digital temperature controller with a display for real-time monitoring and adjustment of temperature settings.

In some embodiments, the heating system may include shock resistance, water resistance, and low power consumption to enhance its durability and usability in rugged field environments.

In some embodiments, the heating block may be part of a fully integrated mobile diagnostic system, combining temperature control, sample preparation, and real-time detection within a single compact unit, further enhancing its utility for rapid, on-site pathogen detection.

In a third aspect, there is provided a method of detecting the presence or absence of a *Beta spp.* specific, preferably a *Beta vulgaris* specific, pathogen, suitable for use in a loop-mediated isothermal amplification (LAMP) assay, preferably for the simultaneously detecting at least two *Beta spp.* specific, preferably a *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola,* the method comprising: (i) obtaining and thus providing a sample potentially comprising genetic information of at least one *Beta spp.* specific, preferably a *Beta vulgaris* specific, pathogen; (ii) optionally: mechanically pre-treating the sample; (iii) conducting a LAMP assay with said sample using at least one primer set according to the present invention, or using a detection kit according to the present invention; (iv) obtaining and optionally evaluating at least one test result from the LAMP assay.

One embodiment, there is provided a method, the method further comprising the steps of; (v) identifying at least one *Beta spp.* plant, preferably a *Beta vulgaris* plant tested positive for at least one *Beta spp.* specific pathogen; and (vi) optionally: identifying and optionally applying a pathogen specific treatment. environment.

In some embodiments, the method may further include quantifying the detected pathogen load using fluorescence or colorimetric-based LAMP signal intensities, providing an estimation of infection severity. The pathogen load data may be used to determine the urgency and extent of treatment application.

In some embodiments, the method may involve cross-referencing test results with environmental and agronomic data, such as weather conditions, soil health parameters, and recent pest activity, to predict disease progression and optimize treatment strategies.

In some embodiments, the method may include selecting an appropriate treatment based on the detected pathogen, wherein the treatment may involve chemical, biological, or agronomic interventions. For viral infections, treatment strategies may preferably focus on vector control (e.g., insecticides, biological control agents, or habitat modification), whereas fungal pathogen management may preferably involve fungicidal treatments, resistant variety selection, or cultural practices such as crop rotation, but not limited thereof.

In another embodiment, there is provided a method, wherein the method is at least partially, preferably completely, conducted *in situ* or in a non-laboratory environment.

In some embodiments, the method may be conducted entirely or partially in non-laboratory environments, such as agricultural fields, greenhouses, or storage facilities, allowing for real-time decision-making in disease management. The simplicity and portability of the detection kit may enable testing by personnel with minimal technical expertise, reducing reliance on centralized laboratory facilities.

In one embodiment, there is provided a method, wherein the method is a multiplexing isothermal detection method for detecting at least two, preferably at least three, more preferably for or more than four *Beta spp.* specific, preferably *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola.*

In some embodiments, the multiplexing detection method is designed to simultaneously amplify and detect multiple pathogen targets within a single reaction, significantly reducing assay time and resource consumption compared to single-target detection methods.

In some embodiments, the method may utilize pathogen-specific primer sets labeled with distinct fluorescent dyes or colorimetric indicators, allowing differentiation of multiple pathogens in a single reaction through visual readout or fluorescence detection.

In some embodiments, the method may incorporate internal controls, such as plant host genes or non-target viral/fungal markers, to ensure assay reliability and detect potential false negatives caused by poor sample quality or reaction inhibition.

In some embodiments, the method may utilize microfluidic cartridge-based detection, where multiple LAMP reactions are performed in separate wells or chambers within a single device, enabling high-throughput, automated pathogen screening in field or laboratory settings.

In some embodiments, the method may further comprise semi-quantitative or quantitative analysis, where differences in amplification kinetics, fluorescence intensity, or turbidity changes provide an estimate of pathogen load, allowing for improved disease severity assessment.

In some embodiments, the method may be optimized for direct sample testing, requiring minimal sample preparation steps, such as crude extract lysis, without the need for complex nucleic acid purification, making it highly suitable for in-field applications.

One embodiment relates to the method according to the third aspect or any embodiment thereof, wherein step (iii) comprises a step of lysis.

In another embodiment, there is provided a method according to the present invention, wherein the at least one test result is available in less than one hour, preferably in less than 45 min, more preferably in less than 30 min and most preferably in less than 15 min.

In a fourth aspect, there is provided the use of at least on primer set according to the various aspects and embodiments of the present invention and/or a use of a detection kit according to the various aspects and embodiments of the present invention for detecting the presence or absence of a *Beta spp.* specific, preferably a *Beta vulgaris* specific, pathogen, suitable for use in a loop-mediated isothermal amplification (LAMP) assay, preferably for the simultaneously detecting at least two *Beta spp.* specific, preferably a *Beta vulgaris* specific, pathogens.

One specific embodiment may relate to the use of the present invention, wherein the use is an *in situ* use conducted in a non-laboratory.

### Examples

### Example 1: Detection of Yellowing Viruses Using LAMP Primers

### Material & Methods

### LAMP primer design for BChV, BMYV, BYV and Cercospora beticola

RNA of BChV, BMYV and BYV were extracted from reference infected sugar beet plants growing the KWS insect rearing chamber and the leaf samples from yellowing virus monitoring project 2022 extracted using Quick-RNA^{™} Miniprep Kit (Zymo Research, Freiburg, Germany) according to the manufacturing protocol. DNA of *Cercospora beticola* were extracted from mycelium of the reference isolates of our own KWS culture collection at Department of Phytopathology using (DNeasy Plant Pro Kit, Qiagen, Hilden, Germany). The RNA was transcribed to cDNA using LunaScript^{®} RT Master Mix (New England Biolabs, Ipswich, MA, USA). Genome of BChV, BMYV, BYV and *Cercospora beticola* was partly amplified with PCR and sequenced at Microsynth Seqlab (Göttingen, Germany). Additional sequencing data from BChV, BMYV, BYV and *Cercospora beticola* were also retrieved from NCBI GenBank. All sequencing data from each target were aligned together using ugene software version 46.0. The obtained consensus sequences were used for LAMP primer design (each primer set comprises 6 primers: B3, F3, BIP, FIP, LoopF and LoopB) in LAMP designer software (PREMIER Biosoft, San Francisco, CA, USA) and NEB LAMP design online tool (New England Biolabs, Ipswich, MA, USA). Generated primer sets were prescreening using reference BChV, BYV and BMYV RNA standards produced from infected sugar beet plants growing the KWS insect rearing chambers or *Cercospora beticola* reference DNA standard. The LAMP assays were conducted using WarmStart^{®} Colorimetric LAMP 2X Master Mix (DNA & RNA) (New England Biolabs, Ipswich, MA, USA). The potential primer sets which were specific to BChV (Beet chlorosis virus) were used for further tests for specificity and sensitivity.

### Specificity and sensitivity tests

Three to five high potential primer sets per each targeted pathogens were tested for specificity and sensitivity using standard RNA or DNA dilutions (10⁰ to 10⁻⁶ ng/µl total RNA or DNA) from common viral (BChV, BYV, BMYV and BtMV), bacterial (Candidatus Phytopasma solani and Candidatus Arsenophonus phytopathogenicus) and fungal (*Cercospora* spp. and *Fusarium* spp.) pathogens of sugar beet. The LAMP assays were conducted using WarmStart^{®} Colorimetric LAMP 2X Master Mix (DNA & RNA) (New England Biolabs, Ipswich, MA, USA) according to the manufacturing recommendations. The incubation times were tested at 15, 20, 25, 30, 45, 60 and 90 mins. The sensitivity of detection were compared with the results from the magnetic induction cycler qPCR (Bio Molecular Systems, Upper Coomera, Australia). The primer sets which showed the best results was used for accuracy and repeatability tests.

### Results and Discussion

4 LAMP primer sets were successfully designed and showed sufficient, high or very high potential for detection of yellowing viruses (BMYV (primer set: **BM3**, sufficient)/BYV (primer set: **BY1**, very high)/BChV (primer set: **BC5**, high)) and *C. beticola* (primer set: **Cb_I1**, high) (Fig. 1).

Sequence data from in-house facilities strongly contributed to the achievement of generating the new LAMP primers. The efficient detection threshold (sensitivity) was set at 10 to 100 times lower compared with the standard real-time PCR (i.e. total RNA concentration for real-time PCR 10⁻⁵ ng/µl and for LAMP 10⁻⁴ to 10⁻³ ng/µl).

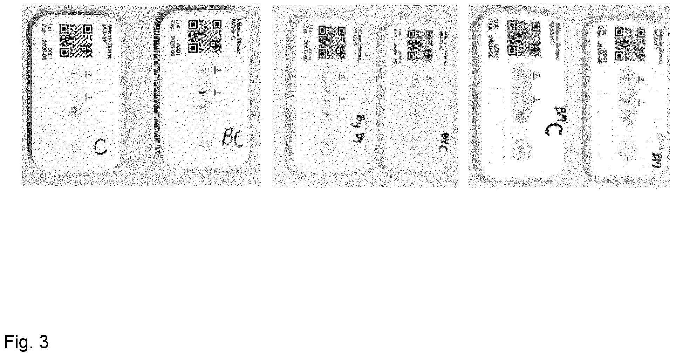

The specificity test included many common sugar beet pathogens encompassing viruses, fungi and bacteria showed that the developed LAMP primers are specific. The primer sets BY1 and Cb_I1 showed stable results with an incubation period of 30 to 45 min (Fig. 3).

The use of LAMP primers for *C. beticola* (Cb_I1) lead to 30 to 45 min (50 to 66.7%) faster results compared to 90 min using the *published* LAMP primers (Shrestha et al., 2020). This was achieved by using 6 LAMP primers for C. *beticola,* by incorporating 2 efficient Loop primers, instead of only 4 LAMP primers according to the published protocol (Shrestha et al., 2020). The BC5 primer set shows stable results after 45 min (Fig. 3).

### Example 2: Detection of Cercospora beticola using LAMP Primers

### Materials & Methods

### Accuracy and Repeatability Test

The accuracy of the BChV, BMYV, and BYV LAMP primers for detecting yellowing viruses in 75 field-collected samples was evaluated by comparing the results with standard protocols using real-time PCR (Mahillon et al., Virus Yellows and Syndrome "Basses Richesses" in Western Switzerland: A Dramatic 2020 Season Calls for Urgent Control Measures, pathogens (2022), 11, 885) for BChV and BYV. Two sensitivity levels of real-time PCR were used in this experiment: 10⁻⁴ ng/µl (100 times less sensitive) and 10⁻³ ng/µl (10 times less sensitive) total RNA. Accuracy tests were also conducted in comparison with standard conventional PCR (Mahillon et al., 2022) and ELISA. The experiment was repeated twice, and the consistency of the results was measured as repeatability (%).

### Results and Discussion

The results for the accuracy rates of the LAMP assays were promising. The accuracy for LAMP BYV was 100% at 10⁻³ ng/µl total RNA and 91.3% at 10⁻⁴ ng/µl total RNA. For LAMP BChV, the accuracy was 98.7% at 10⁻³ ng/µl total RNA and 86% at 10⁻⁴ ng/µl total RNA. LAMP BMYV showed an accuracy of 88% at 10⁻³ ng/µl total RNA (Table 1 and accuracy and repeatability Excel file). The accuracy of LAMP assays with the newly developed LAMP primers was significantly higher than that of conventional PCR (24-82%) and ELISA (25% for BYV). **Table 1** shows comparisons of accuracy rates (as compared with real-time PCR at 10⁻³ ng/µl plant-virus mixed RNA, 100 time less sensitive) obtained by LAMP (with new primer sets), conventional PCR and ELISA (original data are provided in excel file). NA = not accessible.

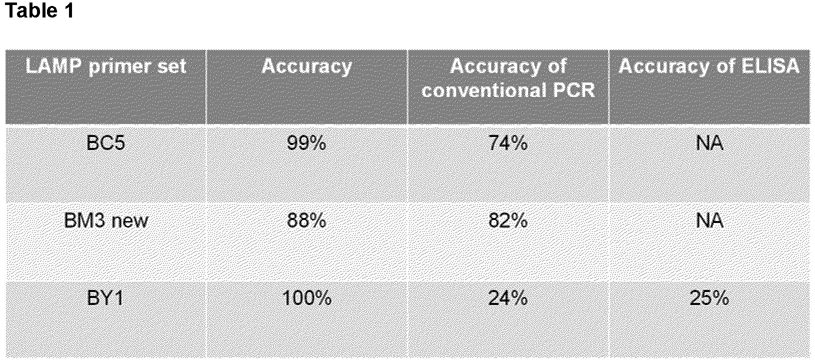

It should be noted that ELISA cannot differentiate between BChV and BMYV, so this test was not performed for these viruses. The repeatability rates of the LAMP assays ranged from 83% to 89%.

Repeatability could be improved by performing two technical replicates for each test. However, most of the uncertain samples had low to very low viral concentrations.

### Example 3: Validation of LyoPrime WarmStart fluorescent LAMP/RT-LAMP Mix (with UDG) (New England Biolabs, UK)

The aims of this experiment were to test and validate the LyoPrime WarmStart fluorescent LAMP/RT-LAMP Mix (with UDG) for detection of three important yellowing viruses in sugar beet (Beet yellows virus (BYV), Beet mild yellowing virus (BMYV) and Beet chlorosis virus (BChV)) and Cercospora using the established LAMP primers (BC5 for BChV, BM3 for BMYV, BY1 for BYV and CBI1 for Cercospora). The advantages of LyoPrime WarmStart fluorescent LAMP/RT-LAMP Mix (with UDG) are (i) it can be kept at room temperature, (ii) it can be mixed with primers during the lyophilized process and (iii) it can be detected also with real-time PCR machine. Moreover, when the LAMP assays are used together with the lateral flow assays, the color indicator is unnecessary.

### Material and methods

Nineteen RNA samples with known viral concentrations (determined by RT-qPCR) according to the KWS standard protocol were used. One PCR water sample was included as a negative control. LAMP assays were prepared in a similar manner to the protocols from the WarmStart^{®} Colorimetric LAMP 2X Master Mix (DNA & RNA), but using LyoPrime WarmStart fluorescent LAMP/RT-LAMP Mix (with UDG) instead (see the general LAMP assay procedure below). The LAMP reaction volume was 25 µL, and the incubation time ranged from 20 to 25 minutes at 65°C. A portable heat block was used. After incubation, the LAMP product was analyzed using the Milenia HybriDetect, and results were recorded after 5 minutes.

Two sets of leaf samples (leaf discs with a 5 mm diameter containing 0, 1, or 2 lesions of Cercospora-infected leaves) were used. An additional sample that tested negative for *Cercospora* was included as a negative control. LAMP assays were prepared in a similar manner to the protocols from the WarmStart^{®} Colorimetric LAMP 2X Master Mix (DNA & RNA), but using LyoPrime WarmStart fluorescent LAMP/RT-LAMP Mix (with UDG) instead. The LAMP reaction volume was 25 µL, and the incubation time was up to 30 minutes at 65°C. A portable heat block was used. After incubation, the LAMP product was analyzed using the Milenia HybriDetect, and results were recorded after 5 minutes. The portable heat block was used. After the incubation, the LAMP product was analyzed with the Milenia HybriDetect and the results were recorded after 5 mins.

### General LAMP assay procedure

1. LAMP solution preparation: Transfer the contents of tube 2 (primers) into tube 1, then mix by shaking.
2. DNA/RNA extraction using the metal ball system and wash buffer: Place the sample in tube 3 along with the metal ball, then shake. Use paper to collect DNA/RNA by pressing it into the sample six times, then wash in buffer 4 30 times.

Alternatively:
2. DNA/RNA extraction using the bag system and wash buffer: Place the sample in a bag and add lysis buffer from tube 3. Grind the sample using a pen, then use paper to collect DNA/RNA six times, followed by washing in buffer 4 30 times.
3. Insert the paper into tube 1, repeat 15 times, then place tube 1 in an incubator at 65°C for 20 minutes (up to 30 minutes if needed).
4. Lateral flow detection: Prepare 150 µL of lateral flow buffer (solution 5) in a 200 µL strip. Add solution 5 to solution 1, then transfer all liquid onto the lateral flow test strip.
5. Read and record the results after 5 minutes.

### Results and discussion

The results demonstrate the successful validation of LAMP tests using the LyoPrime WarmStart fluorescent LAMP/RT-LAMP Mix (with UDG) (Fig. 3). A high level of consistency was observed between LAMP tests based on LyoPrime WarmStart fluorescent LAMP/RT-LAMP Mix (with UDG) and qPCR results. **Table 2** shows a comparison of LAMP tests based on LyoPrime WarmStart fluorescent LAMP/RT-LAMP Mix (with UDG) (Lyo) and qPCR results for detection of three yellowing viruses. Green and orange color highlights indicate consistent and inconsistent results of qPCR and LAMP assays, respectively.

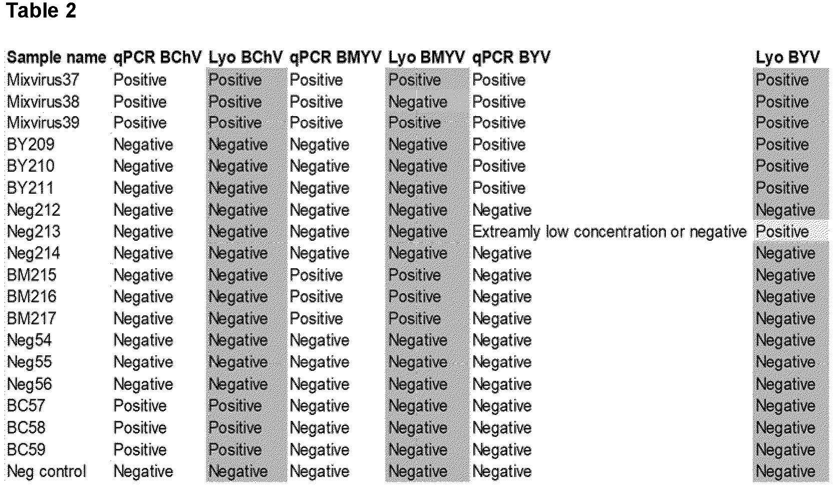

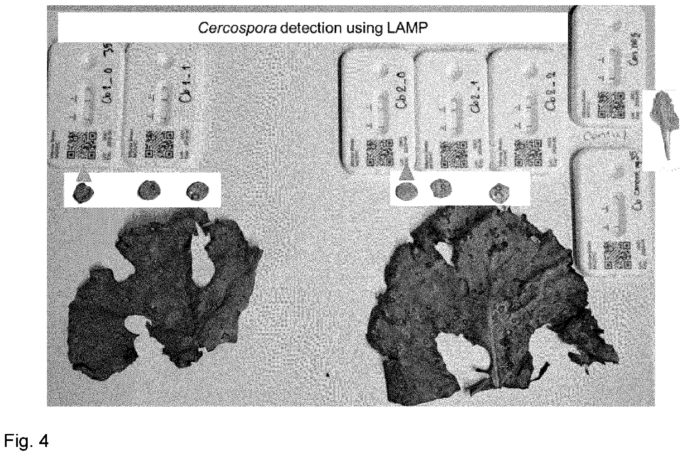

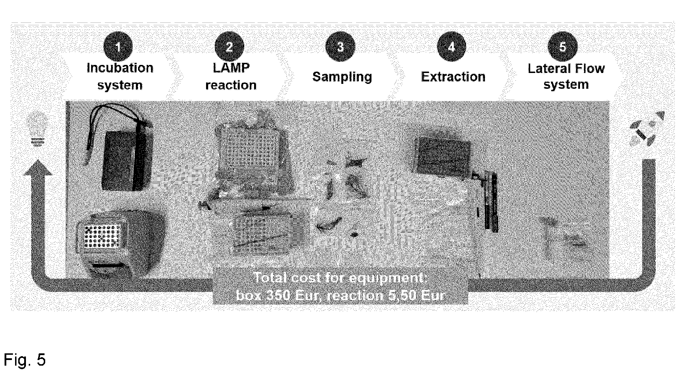

Overall, the findings confirm that the LyoPrime WarmStart fluorescent LAMP/RT-LAMP Mix (with UDG) is compatible with the developed primers and the LAMP assay protocol for detecting yellowing viruses. It can serve as a suitable alternative to the WarmStart^{®} Colorimetric LAMP 2X Master Mix (DNA & RNA). LAMP tests using the LyoPrime WarmStart fluorescent LAMP/RT-LAMP Mix (with UDG) were successfully applied to detect *Cercospora* in all infected sugar beet leaves, including leaf discs containing 0, 1, and 2 lesions of *Cercospora* symptoms (Fig. 4). The negative control remained negative after a 30-minute incubation. These results indicate that LAMP tests based on the LyoPrime WarmStart fluorescent LAMP/RT-LAMP Mix (with UDG) are highly sensitive and can serve as a suitable alternative to the WarmStart^{®} Colorimetric LAMP 2X Master Mix (DNA & RNA). Early detection is achievable with a 30-minute incubation at 65°C. A prototypical LAMP assay toolkit is shown in Fig. 5.

## Claims

1. A primer set for detecting the presence or absence of a *Beta spp.* specific, preferably a *Beta vulgaris* specific, pathogen, suitable for use in a loop-mediated isothermal amplification (LAMP) assay, preferably for the simultaneously detecting at least two *Beta spp.* specific, preferably a *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola,* wherein each of said at least one primer set comprises a forward primer, a reverse primer, a forward inner primer, a reverse inner primer, a loop forward primer and a loop reverse primer, wherein the primer sets are selected from:
(i) a primer set for detecting BChV, comprising one primer each from the six groups selected from
(i1) SEQ ID NO: 1 and 2,
(i2) SEQ ID NO: 3 and 4,
(i3) SEQ ID NO: 5 and 6,
(i4) SEQ ID NO: 7 and 8,
(i5) SEQ ID NO: 9 and 11,
(i6) SEQ ID NO: 12 and 13, and/or
(ii) a primer set for detecting BMYV, comprising one primer each from the six groups selected from
(ii1) SEQ ID NO: 14,
(ii2) SEQ ID NO: 15,
(ii3) SEQ ID NO: 16 and 17,
(ii4) SEQ ID NO: 18 and 19,
(ii5) SEQ ID NO: 20,
(ii6) SEQ ID NO: 21, and/or
(iii) a primer set for detecting BYV, comprising one primer each from the six groups selected from
(iii1) SEQ ID NO: 22 and 23,
(iii2) SEQ ID NO: 24 and 25,
(iii3) SEQ ID NO: 26 and 27,
(iii4) SEQ ID NO: 28 and 29,
(iii5) SEQ ID NO: 30 and 31,
(iii6) SEQ ID NO: 32 and 33, and/or
(iv) a primer set for detecting *Cercospora beticola,* comprising one primer each from the six groups selected from
(iv1) SEQ ID NO: 34 and 35,
(iv2) SEQ ID NO: 36 and 37,
(iv3) SEQ ID NO: 38 and 39,
(iv4) SEQ ID NO: 40 and 41,
(iv5) SEQ ID NO: 42 and 43, and/or
(iv6) SEQ ID NO: 44 and 45.

2. The primer set of claim 1 comprising at least two, three, or all four primer sets of (i), (ii), (iii), or (iv) in combination with each other.

3. The primer set according to claim 1 or 2, wherein the primer set for detecting BChV comprises the set of sequences as defined by SEQ ID NO: 2, 4, 6, 8, 11 and 13, and/or the primer set for detecting BMYV comprises the set of sequences as defined by SEQ ID NO: 14, 15, 17, 18, 20 and 21, and/or the primer set for detecting BYV comprises the set of sequences as defined by SEQ ID NO: 22, 24, 26, 28, 30 and 32, and/or wherein primer set for detecting *Cercospora beticola* comprises the set of sequences as defined by SEQ ID NO: 35, 37, 39, 41, 43 and 45.

4. The primer set according to any of the preceding claims, wherein at least one primer, preferably at least one forward inner or at least one reverse primer, comprises at least one label, preferably a 5'-label, preferably wherein the label is selected from the group of biotin, FITC and FAM.

5. A detection kit for detecting the presence or absence of at least one *Beta spp.* specific, preferably a *Beta vulgaris* specific, pathogen, wherein the kit comprises at least one primer set according to any one of claims 1 to 4, and optionally at least one suitable buffer and/or at least one suitable solution, preferably a lysis buffer.

6. The detection kit according to claim 5, wherein each primer or primer set is contained in one container or compartment in a lyophilized form, or wherein each primer or primer set is composed of a solution or mixed solution.

7. The detection kit according to claim 5, wherein the detection kit is configured for *in situ* use in a field, wherein the kit comprises:
(i) at least one primer set according to any one of claims 1 to 4;
(ii) at least one test cartridge, optionally including at least one sample vial comprising: a reaction chamber or cartridge configured to receive a sample;
(iii) at least one enzyme, optionally immobilized or provided in or with at least one buffer, for performing a LAMP assay on a sample;
(iv) at least one portable heating system, preferably heat block, wherein the at least one portable heating system achieves a temperature of up to 55°C, preferably up to 60°C, more preferably up to 65°C, most preferably at least 65°C;
(v) optionally: at least one high performance power bank;
(vi) optionally: at least one suitable chemical or mechanical reagent; and
(vii) optionally: at least one micropipette.

8. A method of detecting the presence or absence of a *Beta spp.* specific, preferably a *Beta vulgaris* specific, pathogen, suitable for use in a loop-mediated isothermal amplification (LAMP) assay, preferably for the simultaneously detecting at least two *Beta spp.* specific, preferably a *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola,* the method comprising:
(i) obtaining and thus providing a sample potentially comprising genetic information of at least one *Beta spp.* specific, preferably a *Beta vulgaris* specific, pathogen;
(ii) optionally: mechanically pre-treating the sample;
(iii) conducting a LAMP assay with said sample using at least one primer set as defined in any one of claims 1 to 4, or using a detection kit as defined in any one of claims 5 to 7;
(iv) obtaining and optionally evaluating at least one test result from the LAMP assay.

9. The method according to claim 8, further comprising the steps of;
(v) identifying at least one *Beta spp.* plant, preferably a *Beta vulgaris* plant tested positive for at least one *Beta spp.* specific pathogen; and
(vi) optionally: identifying and optionally applying a pathogen specific treatment.

10. The method according to claim 8 or 9, wherein the method is at least partially, preferably completely, conducted *in situ* or in a non-laboratory environment.

11. The method according to any one of claims 8 to 10, wherein the method is a multiplexing isothermal detection method for detecting at least two, preferably at least three, more preferably for or more than four *Beta spp.* specific, preferably *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola.*

12. The method according to any one of claims 8 to 11, wherein step (iii) comprises a step of lysis.

13. The method according to any of claims 8 to 12, wherein the at least one test result is available in less than one hour, preferably in less than 45 min, more preferably in less than 30 min and most preferably in less than 15 min.

14. A use of at least on primer set according to any of claims 1 to 4 and/or a use of a detection kit according to any of claims 5 to 7 for detecting the presence or absence of a *Beta spp.* specific, preferably a *Beta vulgaris* specific, pathogen, suitable for use in a loop-mediated isothermal amplification (LAMP) assay, preferably for the simultaneously detecting at least two *Beta spp.* specific, preferably a *Beta vulgaris* specific, pathogens.

15. The use of claim 14, wherein the use is an *in situ* use conducted in a non-laboratory environment.
